# EUROPEAN PATENT APPLICATION

(11) **EP 2 147 699 A2**
(43) Date of publication of application: **27.01.2010**
(21) Application number: 09162712.5
(22) Date of filing: 26.03.2001
(51) Int. Cl.: A63B 21/055, A61F 5/01, A63B 21/008, A63B 23/035

(54) **Exercise device**

(30) Priority: 27.03.2000 US 535845
(62) Divisional of application: 01302793.3
(71) Applicant: Pape, Leslie, 6 Moygannon Road Warren Point Ulster Down BT34 3QJ (GB)
(72) Inventor: Pape, Leslie, 6 Moygannon Road Warren Point Ulster Down BT34 3QJ (GB)
(74) Representative: Burrows, Anthony Gregory

(57) **Abstract**

An exercise device for a person includes mounting straps 12 and 14 for mounting on respective body members on opposite sides of a selected joint of the person, support members 18 and 20 connected to the mounting straps 12 and 14 and a resistance mechanism 65 interconnecting the support members 18 and for exercising at least one of the two body members upon repeated moving of at least one of the members. The selected joint comprises one of a knee, a hip, an ankle, a wrist, an elbow, and a shoulder. The resistance mechanism 65 includes a pulley 64 with an associated rubber band 66,68 connected to the support members 18 and 20.

## Description

This invention relates generally to exercise devices and, more particularly, to such a device worn by a user for flexing the user's upper body muscles, for example, the muscles in the shoulders, hips, upper arms, lower arms, upper legs, lower legs, ankles and wrists.

US-A-4,993,705 discloses an athletic device for the upper body, including a vest, and an elastically expandable strap fastened in place in an X-configuration across a back part of the vest and extended over the user's shoulders to terminate with two lower arm or wrist cuffs.

US-A-4,911,439 discloses a resilient exercise apparatus including a pair of loops made of a single length of elastic cord to form generally figure eight shape, and mounted over the shoulders and around the waist of the user, with a tubular handle mounted on each front and side section of the cord.

US-A-3,162,441 discloses an exercise device including a frame for mounting on the back or chest of a user via upper and lower belts, three springs and a plurality of pulleys on the frame to accommodate a flexible cord with two stirrups.

GB-A-245,274 discloses an exercise device including a belt adapted to be secured around the waist or to be hung over the shoulders of the user, with elastic cords attached via springs to the belt, with hand grips at the ends thereof.

Exercise devices associated with waist belts and hand grips and/or wrist cuffs are shown and described in US-A-5,433,688; US-A-4,685,671; US-A-4,540,173; US-A-4,441,707; US-A-3,999,752; US-A-2,035,010; US-A-1,432,013; and GB-A-20,463/1908.

Each of US-A-5,292,303; US-A-4,271,831 and US-A-5,409,449 discloses orthopaedic braces hinged at the knee joint of the wearer for free but adjustably limited knee movement. US-A-5,316,547 discloses an orthopaedic brace having medial and lateral knee hinges, with pneumatic pads for positioning against the body of a user. US-A-4,768,500 discloses an athletic knee protector with upper and lower leaf spring members interconnected by gear teeth pivotally engaged between a pair of plates.

According to the present invention, there is provided an exercise device for a person, said exercise device comprising first and second mounting straps for mounting on respective body members on opposite sides of a joint of the person, support members connected to the respective mounting straps, and yielding resistance means interconnecting the support members for exercising at least one of the two body members upon relative movement between said body members about said joint, said resistance means comprising an arcuate surface and a continuous elastomeric member secured at the ends thereof to the respective support members and extending over said arcuate surface.

Owing to the invention, it is possible to provide an improved exercise device to be worn by a user to exercise body parts, such as muscles, adjacent his or her shoulder, hip, knee, ankle, elbow and/or wrist joints.

It is also possible to provide an exercise arrangement including a pulley and one or more rubber bands for use adjacent various body joints.

In order that the invention may be clearly and completely disclosed, reference will now be made, by way of example, to the accompanying drawings, in which:-
Figures 1 to 5 are side elevations of various, similar exercise devices adapted to being used on oppositely disposed body parts adjacent a knee joint, a hip joint, an ankle joint, a wrist joint, and an elbow joint, respectively, but not in accordance with the present invention;
Figure 6 is a front perspective view of a similar exercise devices adapted to being used on oppositely disposed body parts adjacent a shoulder joint, but not in accordance with the present invention;
Figure 7 is a rear perspective view of the exercise device of Figure 6;
Figure 8 is a perspective view of a lid of a rotary damper of the exercise devices of Figures 1 to 7;
Figure 9 is a perspective view of an impeller of the rotary damper;
Figure 10 is a perspective view of a base of the rotary damper;
Figure 11 is a perspective view of an impeller of an alternate rotary damper;
Figure 12 is a perspective view of a base of the alternate rotary damper; and
Figures 13 to 18 are views corresponding to Figures 1 to 6, respectively, of various, alternate, similar, exercise devices adapted to being used on oppositely disposed body parts adjacent a knee joint, a hip joint, an ankle joint, a wrist joint, an elbow joint, and a shoulder joint, respectively, and all in accordance with the present invention.

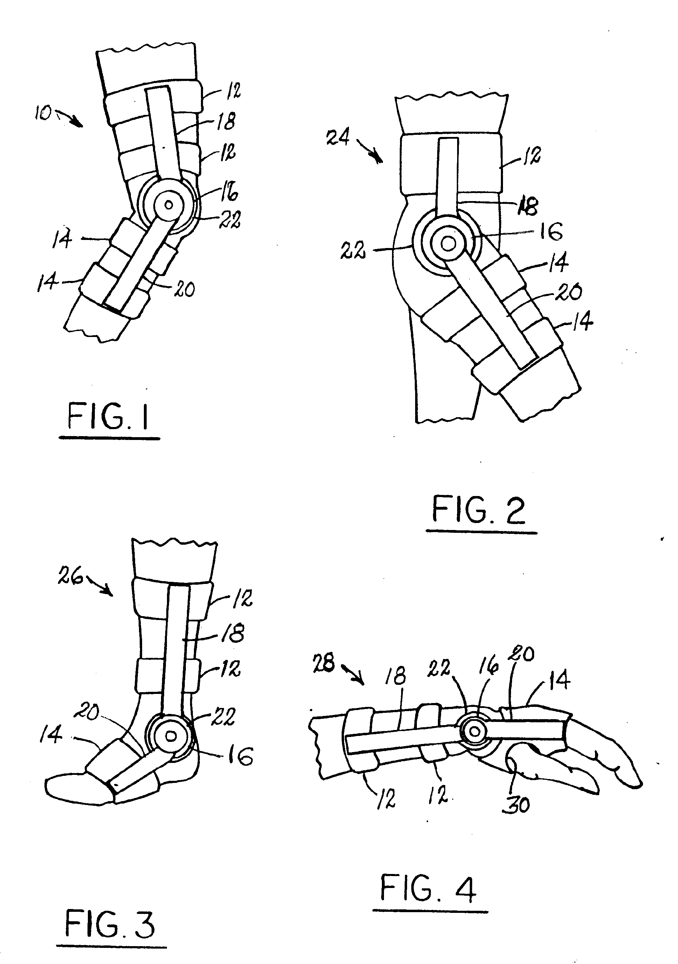

Referring now to the drawings in greater detail, Figure 1 illustrates an exercise device 10 for use on a user's leg, including a pair of spaced-apart upper mounting straps 12 (or a single mounting strap), secured around the leg above the knee joint by any suitable means, such as buckle, snap connector, or Velcro fastening (not shown); a pair of spaced-apart lower mounting straps 14 (or a single mounting strap) secured around the leg below the knee joint; a resistance mechanism in the form of a damper 16 adjacent the knee joint; an upper swing arm 18 attached to both upper straps 12 and to the damper 16; a lower swing arm 20 attached to both lower straps 14 and to the damper 16; and a cushion pad 22 secured to the inner surface of the damper 16 so as to be positioned between the damper 16 and the knee. As will be explained, the damper 16 serves to provide a yielding resistance to each repeated bending and straightening movement about the knee joint.

In Figure 2, an exercise device 24 includes the same elements as in Figure 1, but with a single upper strap 12 mounted around a user's waist and a pair of straps 14 (or only one strap) around an upper leg portion, so as to position the damper 16 and the cushion pad 22 adjacent a hip joint.

In Figure 3, an exercise device 26 likewise includes the same elements as in Figure 1, but mounted around a user's ankle, with a single mounting strap 14 around the user's foot, and with the damper 16 positioned adjacent an ankle joint.

In Figure 4, an exercise device 28 includes a pair of mounting straps 12 around the lower arm and a contoured strap 14 with a thumb hole 30 formed therein and mounted around a user's hand with the damper 16 and cushion pad 22 positioned adjacent the wrist joint.

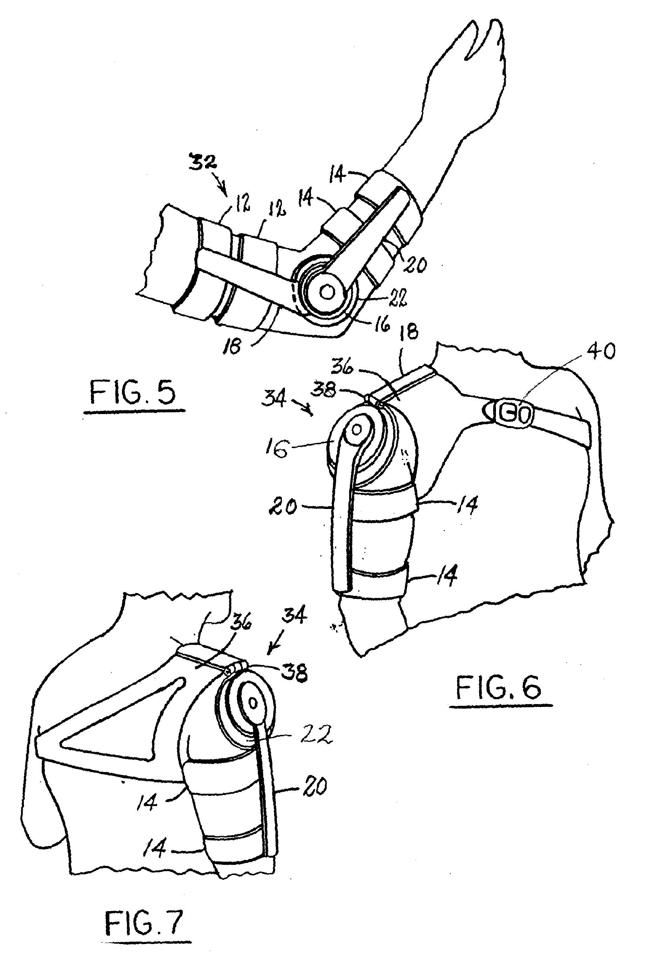

In Figure 5, an exercise device 32 is similar to the Figure 1 exercise device 10 but mounted relative to an elbow joint.

Figures 6 and 7 show an exercise device 34 adaptable to a shoulder joint. The device 34 includes a right- (or left-) hand shoulder strap 36 wrapped around the wearer's back, chest and opposite armpit, connected together on the chest by any suitable means, such as a snap buckle 40. The upper swing arm 18 is secured to the shoulder strap 36 along the shoulder, with the outer end thereof connected by a suitable hinge 38 to an edge of the damper 16 so as to be turnable about an axis transverse to the joint axis. The lower swing arm 20 extends downwardly from an outer surface of the damper 16 to the two lower support straps 14 around the user's upper arm. The cushion pad 22 is between the damper 16 and the upper arm surface adjacent the shoulder.

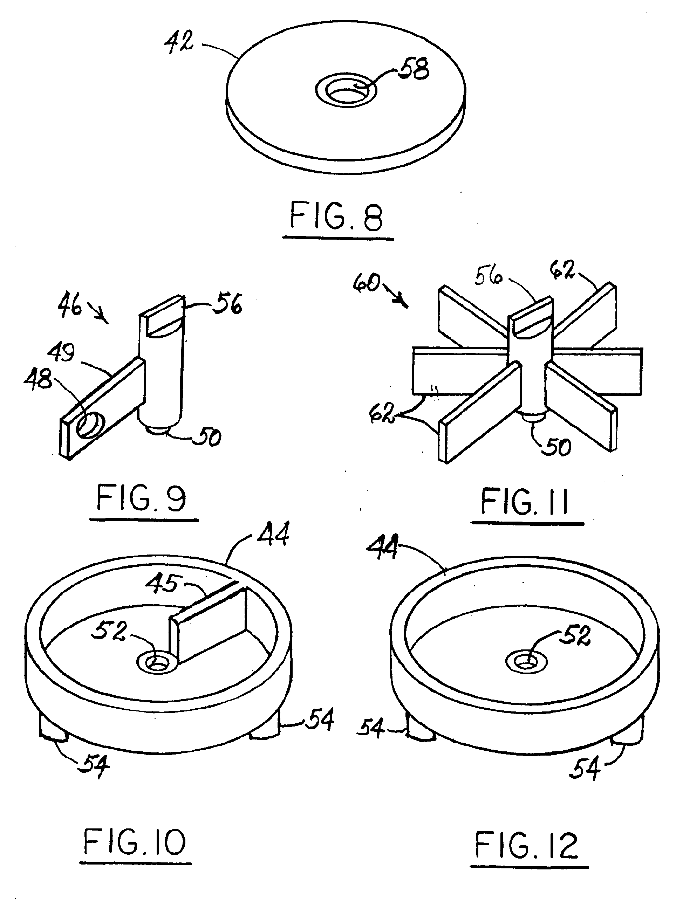

Referring now to Figures 8 to 10, there are shown three elements which form the damper 16. Specifically, the damper 16 includes a lid 42 for mounting on a base 44 including a radially extending inner wall 45, and enclosing an impeller 46 which has a throttling opening 48 of a predetermined throughflow cross-sectional area and formed in a radially outwardly extending wall in the form of a vane 49. The vane 49 is turnable in a selected viscous fluid (not shown) such as a silicone of a predetermined viscosity and, with the wall 45, divides the interior of the damper 16 into two chambers intercommunicating by way of the opening 48. The viscosity of the fluid and/or the opening size in the vane may be varied to increase or decrease the damping torque in co operation with the back and forth flow of the fluid through the opening 48, the oscillation of the impeller 46 being limited only by the engagement of the vane 49 against a side of the wall 45. One formed end 50 of the impeller 46 is turnably mounted in an impeller bearing hole 52 formed in the bottom inner surface of the base 44. Location lugs 54 on an outer surface of the base 44 are secured in any suitable manner to one end of the upper or lower swing arm 18 or 20. A location connector 56 is formed at the other end of the impeller to extend through a sealed bearing hole 58 formed in the centre of the lid 42, for connection in any suitable manner to one end of the lower or upper swing arm 20 or 18 of each assembly.

As shown in Figures 11 and 12, an impeller 60 includes six vanes 62 in lieu of the perforated vane 49, and a base 44 with no inner wall 45. The vanes 62 and the inside diameter of the base 44 are each sized as desired to increase or decrease the rotary damping torque.

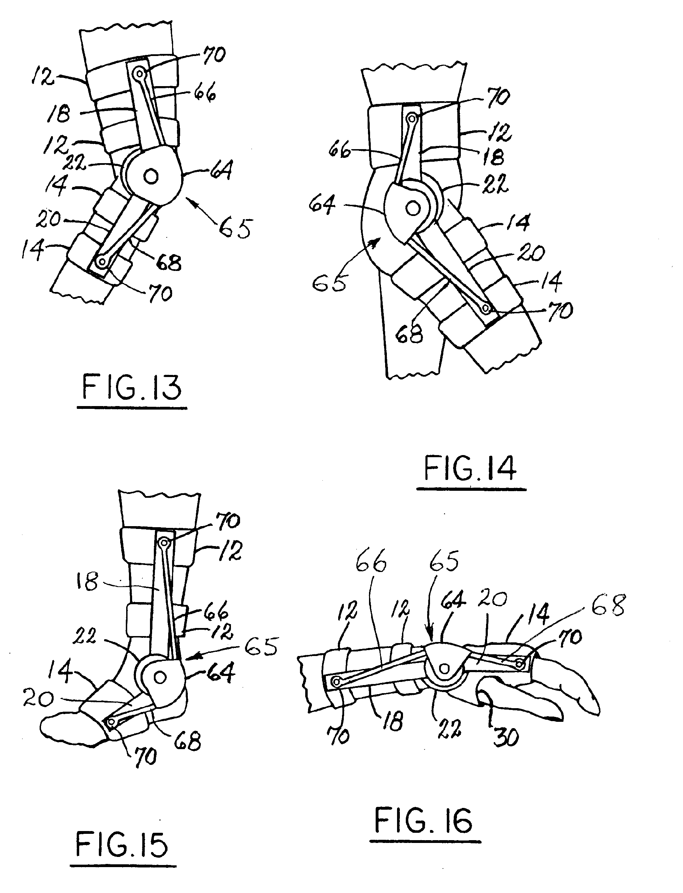

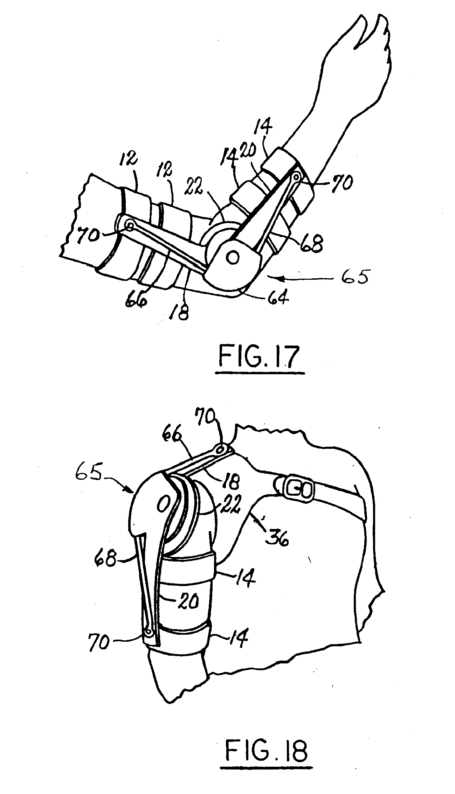

Figures 13, 14, 15, 16, 17 and 18 are comparable to Figures 1, 2, 3, 4, 5 and 6, respectively, except that the resistance mechanism in the form of the damper 16 is replaced with a resistance mechanism 65 comprised of a covered pulley 64 or other covered, arcuate- shaped element having an elastomeric member, such as a rubber band having oppositely disposed end portions 66 and 68, extending therearound and along respective upper and lower swing arms 18 and 20, and having its ends connected to the respective ends of the arms 18 and 20 by suitable studs 70. The pulley 64 may be a simple pulley wheel, with the rubber band 66, 68 looped around a portion thereof. Alternately, the rubber band 66,68 may simply slide along a fixed, arcuate-shaped surface.

In operation, relative movements of the shoulder and upper arm, upper and lower arm, lower arm and hand, upper and lower leg, or lower leg and foot, with the exercise device in place, serve to exercise these body members and their associated joints, so that virtually every muscle group adjacent all body joints can be exercised.

A particular advantage of the exercise devices described with reference to the drawings is that they are compact, without any significant projections away from the body parts, and so can be worn under ordinary clothing and used in everyday life. Thus, they are particularly convenient for use as everyday fitness exercise devices or physiotherapy exercise devices.

## Claims

1. An exercise device for a person, said exercise device comprising first and second mounting straps (12,14) for mounting on respective body members on opposite sides of a joint of the person, support members (18,20) connected to the respective mounting straps (12,14), and yielding resistance means (65) interconnecting the support members (18,20) for exercising at least one of the two body members upon relative movement between said body members about said joint, said resistance means (65) comprising an arcuate surface (64) and a continuous elastomeric member (66,68) secured at the ends thereof to the respective support members (18,20) and extending over said arcuate surface (64).

2. A device according to claim 1, wherein said elastomeric member (66,68) is a rubber band (66,68) connected to the outer ends of the support members (18,20) by respective fasteners (70).

3. A device according to claim 1 or 2, wherein each mounting strap (12,14) is connected to only one support member (18,20).

4. A device according to any preceding claim, wherein said joint is a shoulder joint and an upper one (18) of said support members (18,20) is connected to said resistance means (16;65) so as to be turnable about an axis transverse to the joint axis.
